# EUROPEAN PATENT SPECIFICATION

(11) **EP 4 483 854 B1**
(45) Date of publication and mention of the grant of the patent: **05.08.2026**
(21) Application number: 23182905.2
(22) Date of filing: 30.06.2023
(51) Int. Cl.: A61F 13/42

(54) **DETECTION DEVICE AND CHARGING STATION**
DETEKTIONSVORRICHTUNG UND LADESTATION
DISPOSITIF DE DÉTECTION ET STATION DE CHARGE

(43) Date of publication of application: 01.01.2025
(73) Proprietor: Ontex BV, 9255 Buggenhout (BE); Ontex Group NV, 9320 Erembodegem (BE)
(72) Inventor: Hellmold, Jens, 59269 Beckum (DE); Pepermans, Manu, 2018 Antwerpen (BE)
(74) Representative: Macchetta, Andrea

(56) References cited:
- US-A1- 2004 207 530
- US-B2- 8 933 292
- US-B2- 9 904 562

## Description

### TECHNICAL FIELD

The disclosure relates to detection devices suitable for use in absorbent articles such as baby or adult diapers, pants, or incontinence briefs for adults and/or children. Typically said absorbent articles comprising added electronics adapted to monitor voiding events such as exudate gushes within said absorbent articles. The disclosure further relates to a charging station for charging said detection devices.

### BACKGROUND

Absorbent articles for personal hygiene are designed to absorb and contain bodily exudates, such as a large quantity of urine. Non-limiting examples of disposable absorbent articles include diapers, pants, training pants, pads, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, amongst other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels. Relevant prior art is also represented by US8933292B2.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988. In all such disclosures, electronic components are printed on the backsheet of the absorbent article and various pocket arrangements are described to connect the detection device or clip-on unit to the absorbent article. Nevertheless, they fail to describe a unit especially designed for seamlessly securing itself to the absorbent article in such a way to limit dislodging thereof. Moreover, they fail to describe means that allow to control the position of a unit versus electronic components of the absorbent article in an effective and user-friendly manner.

A need exists for an inexpensive and/or more simple and effective detection means that can be joined to absorbent articles whilst allowing easy reading of information from, for example, a screen thereof and limiting accidental dislodging thereof during wearer's movements. Moreover, a need exists for a design that is compact and easy to insert and be retained into a pocket formed on the absorbent article on a controllable position within such a pocket.

The use of detection devices generates additional costs for absorbent article users. In particular, a detection device contains electronical components such as one or several sensor(s). In addition, disposing large quantities of detection devices comprising electronic components also generates a considerable amount of undesirable waste. There is, hence, a need for a detection device for sensing moisture in an absorbent article and a complementary charging station for charging of the detection devices.

### SUMMARY

In a first aspect, the present invention relates to a detection device comprising: a housing comprising a front face, a back face, and side edges connecting the front and back faces and disposed therebetween, wherein said housing comprises a clip element rotatably connected to the housing; wherein said clip element can be rotated from an open position to a closed position, and wherein said detection device is suitable for coupling to an absorbent article and arranged such that when in said closed position at least one layer of said absorbent article is interposed between said housing and said clip element,
wherein said housing comprises at least one or more non-contact sensor(s) arranged to automatically trigger a detection signal according to whether the clip element is in closed or open position.

In a second aspect, the present invention relates to a charging station, for charging the detection device of the first aspec, comprising: a housing comprising a front face, a back face, a top face, a bottom face and side edges;
wherein the housing comprises at least one or more charging slots and at least one or more contact terminals; wherein the charging station has a cross-section that is polygonal in shape forming a plane figure having three or more sides and wherein at least two of said sides together form an angle of at most about 90° and in that said at least two sides are arranged to come into contact with said detection device in an open position to activate charging.

### BRIEF DESCRIPTION OF THE FIGURES

**FIG. 1** depicts an absorbent article and detection device according to an embodiment herein.
**FIG. 2** depicts schematically a portion of an absorbent article and pocket according to an embodiment herein.
**FIG. 3** depicts a detection device according to an embodiment herein.
**FIG. 4** depicts a detection device and charging station according to an embodiment herein.
**FIG. 5** depicts exemplary embodiments of the cross-section of the charging station being polygonal in shape.
**FIG. 6** depicts an exemplary embodiment of the cross-section of the charging station being trapezoidal in shape illustrating the height ratio of the H2 over H1.
**FIG. 7** depicts the working principle of a inductive proximity sensor (a), capacitive proximity sensor (b) and a magnetic proximity sensor (c).

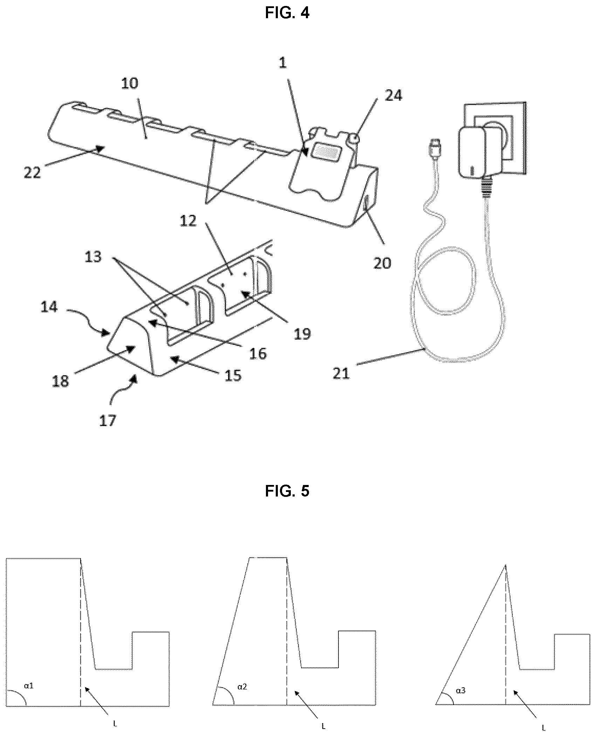

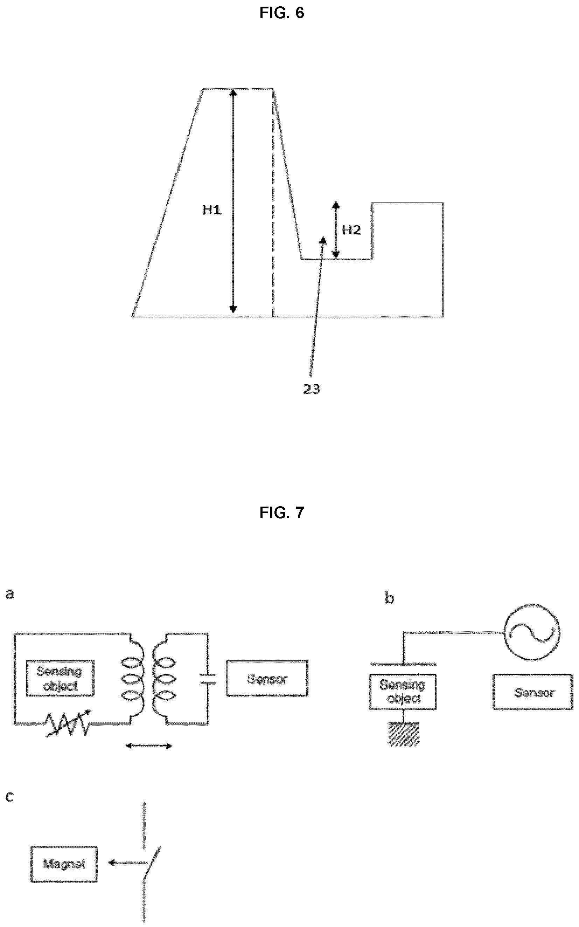

For illustrative purposes, Figs. 5 and 6 show sharp corners but are not limited thereto. The corners of the shown cross-sections can also be rounded corners.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-40% or less, or +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

As used herein, the "body-facing" or "bodyside" or "skin-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The expression "% by weight" or "%wt" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

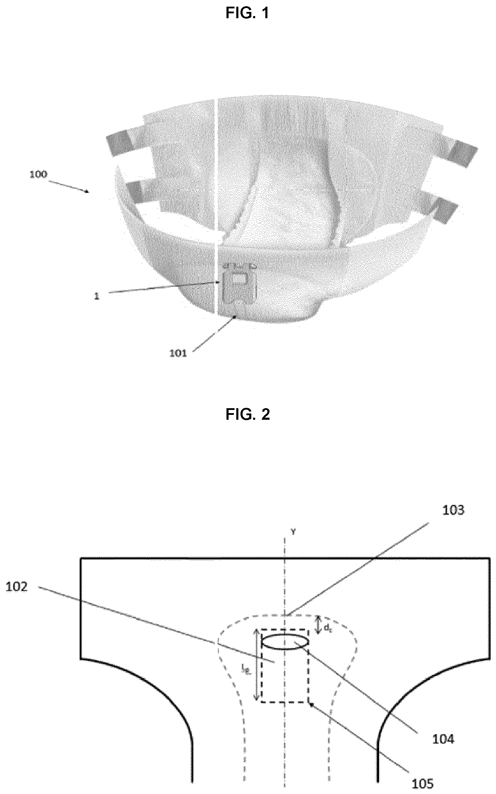

In an embodiment, the absorbent article (100) (as exemplified in Fig. 1) is typically a disposable diaper, pad or pant, and suitable for detecting an exudate and/or wetness (i.e. urine and/or feces) event therein and/or risk of exudate leakage therefrom, said absorbent article comprising: a liquid impermeable backsheet, a liquid permeable topsheet, and an absorbent core interposed between said backsheet and topsheet, wherein said backsheet comprises an exudate monitoring sensor positioned on a body facing surface thereof, preferably said exudate monitoring sensor comprising one or more conductive sensor tracks (101) typically comprising a conductive ink preferably printed onto said body facing surface of the liquid impermeable backsheet.

As exemplified in Fig. 2, the absorbent article (100) may comprise a pocket (102) comprising an opening (104) and a sealed perimeter (105) wherein the body facing surface of the liquid impermeable backsheet is joined to an insulating strip positioned between the absorbent core and said backsheet (typically such that the sensor tracks (101) are interposed between the body facing surface of the backsheet and a garment facing surface of the insulating strip, and arranged such that only said opening (104) is in fluid, typically liquid, communication with the with the rest of said pocket (102), typically said pocket (102) adapted for retaining therein at least a portion of the housing (2) of detection devices described therein may be positioned within a core region, and preferably at a distance d_{c} from a core proximal edge (103), said pocket (102) having a pocket length Lₚ, and wherein d_{c} is from 0.01Lₚ to 0.3Lₚ, preferably from 0.05Lₚ to 0.25Lₚ, more preferably from 0.08Lₚ to 0.20Lₚ. Advantageously such arrangement allows for good comfort and cushioning provided by the core layer through its thickness. By "within a core region" it is meant that the pocket (102) is positioned such that a portion of the core is interposed between the subject and said pocket (102) (and e.g. module when inserted/connected thereto) when the article (100) is being worn.

The article (100) may further comprise a removable detection device (or clip-on data processing module) (1) having a plurality of exposed electrically conductive terminals (8, 8') capable of directly contacting the exudate monitoring sensor so as to be in electrical communication therewith.

In an embodiment, the position for the pocket (102) (i.e. position of detection device attachment) is preferably chosen to ensure good wearing comfort and good accessibility for care givers. The placement is preferably at the front side of the wearer, below a belly button position, so that medical injections to the patient at this point are not hindered. The placement is also not too low and preferably above a pubic bone position to ensure good wearing comfort. The point of attachment is preferably at a position that provides some cushioning and buffering with the absorbent core underneath.

In an embodiment, the absorbent article (100) comprises a liquid permeable topsheet, a liquid impermeable backsheet comprising an opening (104) for the detection device (1) to be inserted therein, and an absorbent core positioned between said topsheet and backsheet, wherein an insulating strip is positioned between said backsheet and said absorbent core forming a pocket (102) adapted to retain the housing (2) of said detection device (1) therein. In a preferred embodiment, the opening (104) has an opening perimeter and the housing (2) has an insertion perimeter, wherein the ratio of the opening perimeter and the insertion perimeter is from 0.9 to 1.3, preferably from 1.0 to 1.2. Preferably wherein the backsheet is arranged to deform (e.g. stretch) upon insertion of the housing (2) through the opening (104). Advantageously this allows to provide a tight fit between the portion of the housing (2) that is inserted into the pocket (102) and the opening (104) which mechanically inhibits dislodging and/or twisting of the device whilst inserting such that it is securely fitted. The absorbent article (100) may further comprise any of the embodiments of the detection device (1) described herein.

### THE DETECTION DEVICE

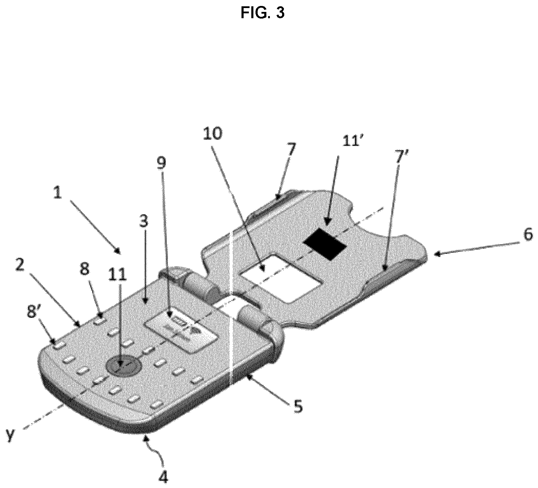

As exemplified in Fig. 3, the detection devices (1) herein comprise: a housing (2) comprising a front face (3), a back face (4), and side edges (5) connecting the front and back faces (3, 4) and disposed therebetween; and a clip element (6) rotatably connected to the housing (2), wherein said clip element (6) can be rotated from an open position to a closed position, and wherein said detection device (1) is suitable for coupling to an absorbent article (100) and arranged such that when in said closed position at one layer of said absorbent article (100) is interposed between said housing (2) and said clip element (6). The housing (2) comprises at least one or more non-contact sensor(s) (11) arranged to automatically trigger a detection signal according to whether the clip element (6) is in closed or open position. Advantageously, this provides for an increased certainty that a detection signal is triggered automatically when the clip element (6) is in closed or open position. Even is the layer of the absorbent article (100) that is interposed between said housing (2) and said clip element (6) contains one or more folds, the use of at least one or more non-contact sensor(s) (11) still allows to recognize that the clip element (6) is in closed position.

In an embodiment, the housing (2) comprises therein a battery, a processor, and a transmitter, and further comprises a plurality of conductive terminals (8, 8') that are exposed on the front face (3) of said housing (2) and facing said clip element (6) when in the closed position, said conductive terminals (8, 8') being arranged to come into contact with one or more conductive sensor tracks (101) positioned on the absorbent article (100), preferably a surface, typically a body facing surface, of the at least one layer of said absorbent article (100) interposed between the housing (2) and the clip element (6) when in the closed position, preferably said layer being a liquid impermeable backsheet of said absorbent article (100). Advantageously this allows for seamless and reliable connection with the conductive tracks (101) of the absorbent article (100) with limited risk of misalignment that would otherwise that would otherwise cause in exudate detection false measurements.

In an embodiment, at least one layer of said absorbent article (100) comprises a liquid impermeable backsheet of said absorbent article (100), preferably consists of a liquid impermeable backsheet and a further liquid permeable nonwoven layer laminated to a garment facing surface of said backsheet. Advantageously, no additional layers or webs need to be added to the absorbent articles (100) in order to connect the detection device (1) with the absorbent article (100).

In an embodiment, the transmitter of the detection device (1) is arranged to transmit signals to the computing means, preferably an algorithm or logic stored on a cloud-based server. The signals can be triggered by one of the following: a change in the state of the absorbent article (101 such as from dry to wet, a first-time connection to an absorbent article (100), and disconnection of the detection device (1) from an absorbent article (100) after a first-time connection to the absorbent article (100)

In an embodiment, the detection signal triggered when the clip element (6) is in closed or open position powers-on the detection device (1) and/or activates the transmitter. Advantageously, this provides for an increased certainty that a detection signal is triggered automatically when the clip element (6) is in closed or open position. Even is the layer of the absorbent article (100) that is interposed between said housing (2) and said clip element (6) contains one or more folds, the use of at least one or more non-contact sensor(s) (11) still allows to recognize that the clip element (6) is in closed position.

In an embodiment, the detection device (1) preferably comprises more than 4 conductive terminals (8, 8'), more preferably more than 5 conductive terminals (8, 8'), even more preferably from 6 to 20 conductive terminals (8,8'), and wherein said conductive terminals (8, 8') are arranged in two or more rows along a length of the front face (3) of the housing (2) typically extending parallel to a longitudinal axis (y). This arrangement allows for a plurality of open circuits to be made and thus allow for multiple permutations of detection combinations when connected to the absorbent article (100) that allows for improved flexibility of use. For example, the terminals (8, 8') may also be used to determine automatically at least one of the absorbency level, type, and/or size, of an absorbent article (100) when connected thereto and to automatically communicate this information to a caregiver by sending a respective signal to a communication device such as a mobile device.

In an embodiment, the conductive terminals (8, 8') of the detection device (1) preferably comprise metal parts or parts of a highly conductive material, even more preferably ferromagnetic materials. In a preferred embodiment, at least one or more of the conductive terminals (8, 8') are magnetic. Advantageously, this allows the conductive terminals to be attracted to a magnet or magnetic materials as a consequence of their substantial magnetic permeability.

In an embodiment, the clip element (6) is removably connected to the housing (2) typically such that it may be replaced with a new one in case of damage or excessive soiling. The clip element (6), which is externally exposed, is the portion of the detection device (1) that is more prone to damages such as scratches, chips or soiling, advantageously this allows for such component to be easily and quickly removed and replaced when needed.

In a preferred embodiment, the clip element (6) is free of any electronics (such as electric wires, sensors, displays, battery, processors, etc.). Advantageously, this renders the clip element (6) easy and cheap to replace.

In an embodiment, the clip element (6) comprises one or more locking elements (7, 7') arranged to mechanically engage with at least a portion of the side edge (5) (preferably only a portion of said side edge typically said portion extending up to about half of the total depth of said side edge), when in a closed position, and wherein said clip element (6) can be rotated from an open position, where the locking elements (7, 7') do not mechanically engage with said side edge (5), to said closed position, and wherein said detection device (1) is suitable for coupling to an absorbent article (100) and arranged such that when in said closed position at least one layer of said absorbent article (100) is interposed between said housing (2) and said clip element (6). Advantageously, this arrangement allows for a secure fit of the detection device (1) to the absorbent article (100) in an intuitive and cost-effective way without risking to damage and/or tear the backsheet layer that is interposed between the clip element (6) and the housing (2) when the detection device (1) is connected to the absorbent article (100), the interposed layer being beneficial to strongly adhere the detection device (1) to the absorbent article (100) and ensure good electrical communication with the sensor tracks (101) even when a subject moves around. It is undesirable that the locking elements (7, 7') of the clip element (6) mechanically engage with a portion of the back face of the detection device (1) as this could lead to hire chances of tearing.

In an embodiment, the clip element (6) comprises two or more locking elements (7, 7') oppositely disposed on either side of a longitudinal centerline (y) running between said locking elements (7, 7') and being parallel to a longest edge of said clip element (6), and wherein each said locking elements (7, 7') are arranged to fasten onto the housing (2) by mechanically engaging with oppositely disposed side edges (5) of the housing (2). Preferably, the locking elements (7, 7') are free of sharp edges and comprise one or more rounded protrusions. Advantageously this allows for a secure fit when one or more layers of the absorbent article (100) are to be positioned between the housing (2) and the clip element (6), whilst limiting the risk of tearing of said layers as well as secured conductive interconnection upon usage and mechanically secured against unintended removal.

### THE PROXIMITY SENSOR

The term "Proximity Sensor" encompasses all sensors that perform non-contact detection in comparison to sensors which detect objects by physically contacting them. Proximity sensors convert information on the movement or presence of an object into an electrical signal.

In a preferred embodiment, the at least one or more non-contact sensor(s) (11, 11') are selected from more of the following: inductive proximity sensor, capacitive proximity sensor, photoelectric proximity sensor, magnetic proximity sensor, ultrasonic proximity sensor.

In an embodiment, the detection device (1) comprises at least one inductive proximity sensor. An inductive proximity sensor detects magnetic loss due to Eddy currents that are generated on a conductive surface by an external magnetic field. The sensing object and inductive proximity sensor form what appears to be a transformer-like relationship as exemplified in Fig. 7(a). An inductive proximity sensor typically features an oscillator whose windings constitute the sensing face and generates an electromagnetic field. The electromagnetic field is generated within the vicinity of the sensing face to create a detection zone. Advantageously, the use of at least one inductive proximity sensor provides for high accuracy, fast response and resistance to harsh industrial environments.

In an embodiment, the detection device (1) comprises at least one capacitive proximity sensor. A capacitive proximity sensor detects changes in the capacitance or electrical charge between the sensing object and the sensor. The amount of capacitance varies depending on the size and distance of the sensing object. An ordinary Capacitive Proximity Sensor is similar to a capacitor with two parallel plates, where the capacity of the two plates is detected. One of the plates is the object being measured (with an imaginary ground), and the other is the Sensor's sensing surface. The changes in the capacity generated between these two poles are detected as exemplified in Fig. 7(b). The objects that can be detected depend on their dielectric constant, but they include resin and water in addition to metals. The dielectric separates the two parallel plates of the capacitor and simply increases the ability of the capacitor to hold electrical charges. Advantageously, the use of at least one capacitive proximity sensor offers higher flexibility by the possibility to detect metallic, non-metallic and liquid targets. They are also highly accurate and repeatable while offering a long operational lifespan.

In an embodiment, the detection device (1) comprises at least one magnetic proximity sensor. As exemplified in Fig. 7(c), a typical magnetic proximity sensor features two ferromagnetic reeds (or contact blades) sealed in a glass casing filled with inert gas. The reed end of the switch is operated by a magnet. When the reed switch is turned ON, the Sensor is turned ON. Advantageously, magnetic proximity switches are ideal for detecting ferromagnetic targets. In addition, their glass tube encasing ensures they are protected from corrosion.

In a preferred embodiment, at least one or more non-contact sensor(s) (11, 11') emits a field of energy and detects the presence of a target (11') when said target enters said field. The emitted field of energy depends on the type of proximity sensor and is selected from one of: sound, light,infrared radiation (IR), or electromagnetic fields.

In an embodiment, as exemplified in Fig. 3, the proximity sensor (11) is placed on the housing (2) whereas the sensing object (11') is placed on the clip element (6). In an alternative embodiment, the proximity sensor (11) is placed on the clip element (6) and the sensing object (11') is placed on the housing (2).

### THE CHARGING STATION

The charging station (10), as exemplified in Figs. 4-5, is configured to charge the detection devices (1) according to the present invention and may be specifically designed to work together with any aspect/embodiment of a detection device (1) described in this disclosure. In other words, the charging station (10) and the detection devices (1) are "compatible" (i.e., they are configured to work together). One of the advantages of the combination in accordance with the present disclosure is that the detection devices (1) arereusable in an convenient manner. It is possible to recharge the detection devices (1). This may lead to a reduction of (electronic) waste, may therefore be environment-friendly, while it may even lower the costs for users.

The charging station (10), comprises a housing (22) comprising a front face (14), a back face (15), a top face (16), a bottom face (17) and side edges (18). The housing (22) comprises at least one or more charging slots (12) and at least one or more contact terminals (13). The charging station (10) has a cross-section that is polygonal in shape forming a plane figure having three or more sides and wherein at least two of said sides together form an angle (α1, α2, α3) of at most about 90° and in that at least one of the sides is arranged to come into contact with said detection device(s) (1) in an open position to activate charging. Preferably, the at least one of the sides coming into contact with the detection device(s) is selected from the front face (14) or top face (16) of the charging station (10). Advantageously, such a cross-section allows the clip elements (6) to be laid on the resting area, being the top face (16) and/or front face (14) of the charging station (10) while the detection device (1) is charging in one of the charging slots (12).

In an embodiment, the shape of the cross-section of the charging station is selected from any one of the following: triangular, trapezoidal, rectangular. In a preferred embodiment, the shape of the cross-section formed by the front face (14), top face (16), bottom face (17) and an imaginary line (L) is selected from any one of the following: triangular, trapezoidal, rectangular. The angle between the front face (14) and the bottom face (17) of the charging station (10) is between about 15° and about 90°, preferably between about 30° and about 90°, even more preferably between about 45° and about 75°. Advantageously, such a cross-section allows the clip element to be laid on the resting area, being the top face (16) and/or front face (14) of the charging station (10) while the detection device (1) is charging in one of the charging slots (12).

In an embodiment, the at least one or more charging slots (12) comprise a first surface (19) on which the at least one or more contact terminals (13) are positioned, said contact terminals (13) being arranged to come into contact with one or more conductive terminals (101) of the detection device (1). In a preferred embodiment, at least two contact terminals (13) are positioned on the first surface (19) and are arranged to come into contact with the conductive terminals (101) of the detection device (1) being closest to the side edges (5) of the detection device (1). Advantageously, this provides for improved guidance for placement of the detection device (1) within a charging slot (12).

In an embodiment, the housing (22) of the charging station (10) comprises between about 2 and about 10 charging slots (12), preferably between about 3 and about 9 charging slots (12), even more preferably between about 5 and about 7 charging slots (12) and most preferably about 6 charging slots (12). Advantageously, this allows to simultaneously charge one or more detection devices (1) and provides for a more efficient flow in monitoring one or more patients.

In an embodiment, at least one or more contact terminals (13) comprises magnetic holding support arranged for correct positioning of the detection devices (1) into the charging slots (12). Preferably, the at least one or more contact terminals (13) comprise of magnetic materials. Advantageously, the use of magnetic materials as the at least one or more contact terminals (13) allows to create the magnetic holding support by attracting at least one or more conductive terminals (8, 8') of the detection device(s) (1). This aids in correctly positioning the detection devices (1) in the charging slots (12).

In a preferred embodiment, the at least one or more charging slots (12) each comprise a receptacle (23) in order to hold the detection devices (1). Furthermore the receptacle has a receptacle height (H2) from about 5 mm to about 50 mm, preferably from about 7 mm to about 45 mm, even more preferably from about 10 mm to about 40 mm, even more preferably from about 10 mm to about 30 mm, even more preferably from about 10 mm to about 25 mm, most preferably from about 10 mm to about 20 mm. Advantageously, in this way sufficient support is provided in order to hold the detection devices (1) within the receptacle (23).

In a preferred embodiment, the at least one or more charging slots (12) each comprise a receptacle (23) in order to hold the detection devices. Furthermore the at least one or more charging slots comprise a ratio between the receptacle height (H2) and the charging station height (H1) between about 0,1 and 1; more preferably between about 0,1 and 0,9; even more preferably between 0,1 and 0,8; even more preferably between about 0,1 and 0,7; even more preferably between about 0,1 and 0,6; even more preferably between about 0,2 and 0,6; even more preferably between about 0,2 and 0,5; even more preferably between about 0,2 and 0,4; most preferably between a 0,25 and 0,35. Advantageously, in this way sufficient support is provided in order to hold the detection devices (1) within the receptable (23) while minimizing material cost and usage for the housing (22) of the charging station (10).

In a preferred embodiment, the housing (2) of the detection device (1) comprises a light source (like an LED) and/or sound source (like a beeper) and/or haptic source (like a buzzer), such signals (or sources) (24) are arranged to give feedback to the caregiver upon attachment to the absorbent article (100) to indicate the charging status for the device (1). In an embodiment, the light source (24) blinks in a certain color, for example green, when the detection device (1) is charging. When charging is complete, the light will be static in the same color, for example static green. Advantageously, this embodiment allows for the caregiver to very easily and intuitively ensure correct alignment of the contact terminals (13) of the charging slots (12) with the terminals (101) of the detection devices (1). This promotes a high user convenience.

In an embodiment, the housing (22) of the charging station (10) further comprises a display adapted to display information relating to the charging status of the inserted detection devices (1), said display being viewable by a caregiver (for example to allow visual verification of the charging status of each detection device (1) and/or charging holder (12).

In a preferred embodiment, the charging station (10) comprises a matching power adapter (21) to supply the charging station (10) of power. Preferably, the power adapter (21) is a USB-C adapter to connect the charging station (10), through a USB-C port (20), to a wall socket for power.

In an embodiment, a kit of parts is provided comprising a plurality of absorbent articles (100) as described herein, a plurality of detection devices (1) as described herein and a charging station (10) containing between about 2 and about 10 charging slots (12), preferably between about 3 and about 9 charging slots (12), even more preferably between about 5 and about 7 charging slots (12) and most preferably about 6 charging slots (12).

It is supposed that the present invention is not restricted to any form of realization described previously and that some modifications can be added to the presented example of fabrication without reappraisal of the appended claims.

## Claims

1. A detection device (1) comprising:
a housing (2) comprising a front face (3), a back face (4), and side edges (5) connecting the front (3) and back (4) faces and disposed therebetween, wherein said housing (2) comprises a clip element (6) rotatably connected to the housing (6); wherein said clip element (6) can be rotated from an open position to a closed position, and wherein said detection device (1) is suitable for coupling to an absorbent article (100) and arranged such that when in said closed position at least one layer of said absorbent article (100) is interposed between said housing (2) and said clip element (6),
**characterized in that** said housing (2) comprises at least one or more non-contact sensor(s) (11, 11') arranged to automatically trigger a detection signal according to whether the clip element (6) is in closed or open position.

2. A detection device (1) according to Claim 1, wherein the detection signal triggers a device-response selected from at least one of: power-on of said detection device (1) and activation of the transmitter.

3. A detection device (1) according to any of the preceding Claims, wherein the at least one or more non-contact sensor(s) (11, 11') are selected from one or more of: inductive proximity sensor, capacitive proximity sensor, photoelectric proximity sensor, magnetic proximity sensor, and ultrasonic proximity sensor.

4. A detection device (1) according to any of the preceding Claims, wherein the at least one or more non-contact sensor(s) (11, 11') emits a field of energy and wherein the presence of a target is detected when said target enters said field.

5. A detection device (1) according to any of the preceding Claims, wherein the housing (2) comprises a plurality of conductive terminals (8, 8') that are exposed on the front face (3) of the housing (2) and facing said clip element (6) when in the closed position said conductive terminals (8, 8') being arranged to come into contact with one or more conductive sensor tracks (101) positioned on the absorbent article (100), preferably a surface, typically a body facing surface, of the at least one layer of said absorbent article (100) interposed between the housing (2) and the clip element (6) when in the closed position, preferably said layer being a liquid impermeable backsheet of said absorbent article (100).

6. A detection device (1) according to Claim 5, wherein the conductive terminals (8, 8') are a plurality, preferably more than 4, more preferably more than 5, even more preferably from 6 to 20, and wherein said conductive terminals (8, 8') are arranged in two or more rows along a length of the front face (3) of the housing (2) typically extending parallel to a longitudinal axis (y).

7. A detection device (1) according to any of Claims 5-6, wherein at least one or more of the conductive terminals (8, 8') are magnetic.

8. A detection device (1) according to any of the preceding Claims, wherein at least one layer of said absorbent article (100) comprises a liquid impermeable backsheet of said absorbent article (100), preferably consists of a said absorbent article (100) comprising a liquid impermeable backsheet and a further liquid permeable nonwoven layer laminated to a garment facing surface of said backsheet.

9. A charging station (10), for charging the detection device(s) of Claim 1, comprising;
a housing (22) comprising a front face (14), a back face (15), a top face (16), a bottom face (17) and side edges (18);
wherein the housing (22) comprises at least one or more charging slots (12) and at least one or more contact terminals (13);
**characterized in that** the charging station (10) has a cross-section that is polygonal in shape forming a plane figure having three or more sides and wherein at least two of said sides together form an angle (α1, α2, α3) of at most about 90° and **in that** at least one of the sides is arranged to come into contact with said detection device(s) (1) in an open position to activate charging and is selected from: front face (14) or top face (16).

10. A charging station (10) according to Claim 9, wherein the at least two sides forming an angle (α1, α2, α3) of at most about 90° are the front face (14) and bottom face (17) and wherein the angle (α1, α2, α3) between the front face (14) and the bottom face (17) of the charging station (10) is between about 15° and about 90°, preferably between about 30° and about 90°, even more preferably between about 45° and about 75°.

11. A charging station (10) according to Claim 10, wherein the at least one or more charging slots (12) comprise a first surface (19) on which the at least one or more contact terminals (13) are positioned, said contact terminals (13) being arranged to come into contact with one or more conductive terminals (8, 8') of the detection device(s) (1).

12. A charging station (10) according to Claim 11, wherein at least two contact terminals (13)are positioned on the first surface (19).

13. A charging station (10) according to Claim 12 wherein the at least two contact terminals (13) are arranged to come into contact with the conductive terminals (8, 8') of the detection device(s) (1) being closest to the side edges (5) of the detection device (1).

14. A charging station (10 according to any of Claims 9 to 13, wherein the housing (22) comprises between about 2 and about 10 charging slots (12), preferably between about 3 and about 9 charging slots (12), even more preferably between about 5 and about 7 charging slots (12) and most preferably about 6 charging slots (12).

15. A charging station (10) according to any of Claims 9 to 14, wherein the at least one or more contact terminals (13) comprise magnetic holding support arranged for correct positioning of the detection device(s) (1) into the charging slots (12).

16. A charging station (10) according to any of Claims 9 to 15, wherein the at least one or more charging slots (12) each comprises a receptacle (23); wherein the receptacle (23) has a receptacle height (H2) from about 5 mm to about 50 mm.

## Patentansprüche

1. Detektionsvorrichtung (1), umfassend:
ein Gehäuse (2), umfassend eine Vorderseite (3), eine Rückseite (4) und Seitenkanten (5), die die Vorder- (3) und Rückseite (4) verbinden und dazwischen platziert sind, wobei das Gehäuse (2) ein Klemmelement (6) umfasst, das mit dem Gehäuse (6) drehbar verbunden ist; wobei das Klemmelement (6) von einer offenen Position in eine geschlossene Position gedreht werden kann, und wobei die Detektionsvorrichtung (1) zum Koppeln mit einem Absorptionsartikel (100) geeignet ist und derart angeordnet ist, dass, wenn es in der geschlossenen Position ist, mindestens eine Lage des Absorptionsartikels (100) zwischen dem Gehäuse (2) und dem Klemmelement (6) eingeschoben ist,
**dadurch gekennzeichnet, dass** das Gehäuse (2) mindestens einen oder mehrere berührungslose Sensor(en) (11, 11') umfasst, die angeordnet sind, um ein Detektionssignal automatisch auszulösen, je nachdem, ob das Klemmelement (6) in geschlossener oder offener Position ist.

2. Detektionsvorrichtung (1) nach Anspruch 1, wobei das Detektionssignal eine Vorrichtungsantwort auslöst, die aus mindestens einem ausgewählt ist von:
Einschalten der Detektionsvorrichtung (1) und Aktivierung des Senders.

3. Detektionsvorrichtung (1) nach einem der vorstehenden Ansprüche, wobei der mindestens eine oder die mehreren berührungslosen Sensor(en) (11, 11') aus einem oder mehreren ausgewählt sind von: induktivem Näherungssensor, kapazitivem Näherungssensor, photoelektrischem Näherungssensor, magnetischem Näherungssensor und Ultraschallnäherungssensor.

4. Detektionsvorrichtung (1) nach einem der vorstehenden Ansprüche, wobei der mindestens eine oder die mehreren berührungslosen Sensor(en) (11, 11') ein Energiefeld aussendet und wobei die Anwesenheit eines Ziels detektiert wird, wenn das Ziel in das Feld eintritt.

5. Detektionsvorrichtung (1) nach einem der vorstehenden Ansprüche, wobei das Gehäuse (2) eine Vielzahl von leitfähigen Anschlüssen (8, 8') umfasst, die an der Vorderseite (3) des Gehäuses (2) frei liegen und dem Klemmelement (6) zugewandt sind, wenn es in der geschlossenen Position ist, wobei die leitfähigen Anschlüsse (8, 8') angeordnet sind, um mit einer oder mehreren leitfähigen Sensorspuren (101) in Kontakt zu kommen, die auf dem Absorptionsartikel (100), vorzugsweise einer Oberfläche, üblicherweise einer dem Körper zugewandten Oberfläche, der mindestens einen Lage des Absorptionsartikels (100) positioniert sind, die zwischen dem Gehäuse (2) und dem Klemmelement (6) eingeschoben ist, wenn es in der geschlossenen Position ist, wobei vorzugsweise die Lage eine flüssigkeitsundurchlässige Rückschicht des Absorptionsartikels (100) ist.

6. Detektionsvorrichtung (1) nach Anspruch 5, wobei die leitfähigen Anschlüsse (8, 8') eine Vielzahl sind, vorzugsweise mehr als 4, mehr bevorzugt mehr als 5, noch mehr bevorzugt von 6 bis 20, und wobei die leitfähigen Anschlüsse (8, 8') in zwei oder mehr Reihen entlang einer Länge der Vorderseite (3) des Gehäuses (2) angeordnet sind, die sich typischerweise parallel zu einer Längsachse (y) erstreckt.

7. Detektionsvorrichtung (1) nach einem der Ansprüche 5 bis 6, wobei mindestens einer oder mehrere der leitfähigen Anschlüsse (8, 8') magnetisch sind.

8. Detektionsvorrichtung (1) nach einem der vorstehenden Ansprüche, wobei mindestens eine Lage des Absorptionsartikels (100) eine flüssigkeitsundurchlässige Rückschicht des Absorptionsartikels (100) umfasst, vorzugsweise aus dem Absorptionsartikel (100) besteht, umfassend eine flüssigkeitsundurchlässige Rückschicht und eine weitere flüssigkeitsdurchlässigen Vliesschicht, die auf eine dem Kleidungsstück zugewandte Oberfläche der Rückschicht laminiert ist.

9. Ladestation (10) zum Laden der Detektionsvorrichtung(en) nach Anspruch 1, umfassend;
ein Gehäuse (22), das eine Vorderseite (14), eine Rückseite (15), eine Oberseite (16), eine Unterseite (17) und Seitenkanten (18) umfasst;
wobei das Gehäuse (22) mindestens einen oder mehrere Ladeschlitze (12) und mindestens einen oder mehrere Kontaktanschlüsse (13) umfasst;
**dadurch gekennzeichnet, dass** die Ladestation (10) einen Querschnitt aufweist, der polygonal geformt ist, der eine Ebenenfigur ausbildet, die drei oder mehr Seiten aufweist, und wobei mindestens zwei der Seiten zusammen einen Winkel (α1, α2, α3) von höchstens etwa 90° ausbilden und **dadurch, dass** mindestens eine der Seiten angeordnet ist, um in einer offenen Position mit der/den Detektionsvorrichtung(en) (1) in Kontakt zu kommen, um das Laden zu aktivieren, und ausgewählt ist aus: Vorderseite (14) oder Oberseite (16).

10. Ladestation (10) nach Anspruch 9, wobei die mindestens zwei Seiten, die einen Winkel (α1, α2, α3) von höchstens etwa 90° ausbilden, die Vorderseite (14) und Unterseite (17) sind und wobei der Winkel (α1, α2, α3) zwischen der Vorderseite (14) und der Bodenseite (17) der Ladestation (10) zwischen etwa 15° und etwa 90°, vorzugsweise zwischen etwa 30° und etwa 90°, noch mehr bevorzugt zwischen etwa 45° und etwa 75° liegt.

11. Ladestation (10) nach Anspruch 10, wobei der mindestens eine oder die mehreren Ladeschlitze (12) eine erste Oberfläche (19) umfassen, auf der der mindestens eine oder die mehreren Kontaktanschlüsse (13) positioniert sind, wobei die Kontaktanschlüsse (13) angeordnet sind, um mit einem oder mehreren leitfähigen Anschlüssen (8, 8') der Detektionsvorrichtung(en) (1) in Kontakt zu kommen.

12. Ladestation (10) nach Anspruch 11, wobei mindestens zwei Kontaktanschlüsse (13) auf der ersten Oberfläche (19) positioniert sind.

13. Ladestation (10) nach Anspruch 12, wobei die mindestens zwei Kontaktanschlüsse (13) angeordnet sind, um mit den leitfähigen Klemmen (8, 8') der Detektionsvorrichtung(en) (1) in Kontakt zu kommen, die den Seitenkanten (5) der Detektionsvorrichtung (1) am nächsten sind.

14. Ladestation (10) nach einem der Ansprüche 9 bis 13, wobei das Gehäuse (22) zwischen etwa 2 und etwa 10 Ladeschlitze (12) umfasst, vorzugsweise zwischen etwa 3 und etwa 9 Ladeschlitze (12), noch mehr bevorzugt zwischen etwa 5 und etwa 7 Ladeschlitze (12) und am meisten bevorzugt etwa 6 Ladeschlitze (12).

15. Ladestation (10) nach einem der Ansprüche 9 bis 14, wobei der mindestens eine oder die mehreren Kontaktanschlüsse (13) eine magnetische Halteunterstützung umfassen, die für eine korrekte Positionierung der Detektionsvorrichtung(en) (1) in den Ladeschlitzen (12) angeordnet ist.

16. Ladestation (10) nach einem der Ansprüche 9 bis 15, wobei der mindestens eine oder die mehreren Ladeschlitze (12) jeweils eine Aufnahme (23) umfassen; wobei die Aufnahme (23) eine Aufnahmehöhe (H2) von etwa 5 mm bis etwa 50 mm aufweist.

## Revendications

1. Dispositif de détection (1) comprenant :
un boîtier (2) comprenant une face avant (3), une face arrière (4), et des bords latéraux (5) reliant les faces avant (3) et arrière (4) et disposés entre elles, dans lequel ledit boîtier (2) comprend un élément de fixation (6) relié au boîtier (2) ; dans lequel ledit élément de fixation (6) peut être tourné d'une position ouverte à une position fermée, et dans lequel ledit dispositif de détection (1) est apte à être accouplé à un article absorbant (100) et agencé de telle sorte que dans ladite position fermée au moins une couche dudit article absorbant (100) est interposée entre ledit boîtier (2) et ledit élément de fixation (6),
**caractérisé en ce que** ledit boîtier (2) comprend au moins un ou plusieurs capteurs sans contact (11, 11') agencés pour déclencher automatiquement un signal de détection selon que l'élément de fixation (6) est en position fermée ou ouverte.

2. Dispositif de détection (1) selon la revendication 1, dans lequel le signal de détection déclenche une réponse de dispositif choisie parmi au moins l'une parmi : la mise sous tension dudit dispositif de détection (1) et l'activation de l'émetteur.

3. Dispositif de détection (1) selon l'une quelconque des revendications précédentes, dans lequel l'au moins un ou plusieurs capteurs sans contact (11, 11') sont choisis parmi un ou plusieurs parmi : capteur de proximité inductif, capteur de proximité capacitif, capteur de proximité photoélectrique, capteur de proximité magnétique, et capteur de proximité à ultrasons.

4. Dispositif de détection (1) selon l'une quelconque des revendications précédentes, dans lequel l'au moins un ou plusieurs capteurs sans contact (11, 11') émettent un champ d'énergie et dans lequel la présence d'une cible est détectée lorsque ladite cible entre dans ledit champ.

5. Dispositif de détection (1) selon l'une quelconque des revendications précédentes, dans lequel le boîtier (2) comprend une pluralité de bornes conductrices (8, 8') qui sont exposées sur la face avant (3) du boîtier (2) et faisant face vers ledit élément de fixation (6) lorsqu'il est dans la position fermée lesdites bornes conductrices (8, 8') étant agencées pour entrer en contact avec une ou plusieurs pistes de capteur conductrices (101) positionnées sur l'article absorbant (100), de préférence une surface, de manière générale une surface faisant face vers le corps, de l'au moins une couche dudit article absorbant (100) intercalée entre le boîtier (2) et l'élément de fixation (6) lorsqu'il est dans la position fermée, de préférence ladite couche étant une feuille arrière imperméable aux liquides dudit article absorbant (100).

6. Dispositif de détection (1) selon la revendication 5, dans lequel les bornes conductrices (8, 8') sont une pluralité, de préférence plus de 4, plus préférablement plus de 5, encore plus préférablement de 6 à 20, et dans lequel lesdites bornes conductrices (8, 8') sont agencées en deux rangées ou plus le long d'une longueur de la face avant (3) du boîtier (2) s'étendant de manière générale parallèlement à un axe longitudinal (y).

7. Dispositif de détection (1) selon l'une quelconque des revendications 5 à 6, dans lequel au moins une ou plusieurs des bornes conductrices (8, 8') sont magnétiques.

8. Dispositif de détection (1) selon l'une quelconque des revendications précédentes, dans lequel au moins une couche dudit article absorbant (100) comprend une feuille arrière imperméable aux liquides dudit article absorbant (100), de préférence se compose d'un dit article absorbant (100) comprenant une feuille arrière imperméable aux liquides et d'une autre couche non tissée perméable aux liquides stratifiée sur une surface faisant face vers le vêtement de ladite feuille arrière.

9. Station de charge (10), permettant de charger le ou les dispositifs de détection selon la revendication 1, comprenant ;
un boîtier (22) comprenant une face avant (14), une face arrière (15), une face supérieure (16), une face inférieure (17) et des bords latéraux (18) ;
dans laquelle le boîtier (22) comprend au moins une ou plusieurs fentes de charge (12) et au moins une ou plusieurs bornes de contact (13) ;
**caractérisée en ce que** la station de charge (10) a une section transversale qui est de forme polygonale formant une figure plane ayant trois côtés ou plus et dans laquelle au moins deux desdits côtés forment ensemble un angle (α1, α2, α3) d'au plus environ 90° et **en ce qu'au** moins l'un des côtés est agencé pour entrer en contact avec ledit ou lesdits dispositifs de détection (1) dans une position ouverte pour activer la charge et est choisi parmi : face avant (14) ou face supérieure (16).

10. Station de charge (10) selon la revendication 9, dans laquelle les au moins deux côtés formant un angle (α1, α2, α3) d'au plus environ 90° sont la face avant (14) et la face inférieure (17) et dans laquelle l'angle (α1, α2, α3) entre la face avant (14) et la face inférieure (17) de la station de charge (10) est compris entre environ 15° et environ 90°, de préférence entre environ 30° et environ 90°, encore plus préférablement entre environ 45° et environ 75°.

11. Station de charge (10) selon la revendication 10, dans laquelle l'au moins une ou plusieurs fentes de charge (12) comprennent une première surface (19) sur laquelle l'au moins une ou plusieurs bornes de contact (13) sont positionnées, lesdites bornes de contact (13) étant agencées pour entrer en contact avec une ou plusieurs bornes conductrices (8, 8') du ou des dispositifs de détection (1).

12. Station de charge (10) selon la revendication 11, dans laquelle au moins deux bornes de contact (13) sont positionnées sur la première surface (19).

13. Station de charge (10) selon la revendication 12, dans laquelle les au moins deux bornes de contact (13) sont agencées pour entrer en contact avec les bornes conductrices (8, 8') du ou des dispositifs de détection (1) étant les plus proches des bords latéraux (5) du dispositif de détection (1).

14. Station de charge (10) selon l'une quelconque des revendications 9 à 13, dans laquelle le boîtier (22) comprend entre environ 2 et environ 10 fentes de charge (12), de préférence entre environ 3 et environ 9 fentes de charge (12), encore plus préférablement entre environ 5 et environ 7 fentes de charge (12) et le plus préférablement environ 6 fentes de charge (12).

15. Station de charge (10) selon l'une quelconque des revendications 9 à 14, dans laquelle l'au moins une ou plusieurs bornes de contact (13) comprennent un support de maintien magnétique agencé pour le positionnement correct du ou des dispositifs de détection (1) dans les fentes de charge (12).

16. Station de charge (10) selon l'une quelconque des revendications 9 à 15, dans laquelle l'au moins une ou plusieurs fentes de charge (12) comprennent chacune un réceptacle (23) ; dans laquelle le réceptacle (23) a une hauteur de réceptacle (H2) d'environ 5 mm à environ 50 mm.
